# EUROPEAN PATENT APPLICATION

(11) **EP 4 751 606 A1**
(43) Date of publication of application: **03.06.2026**
(21) Application number: 24215811.1
(22) Date of filing: 27.11.2024
(51) Int. Cl.: A45D 34/04, A45D 40/26, A45D 40/28, A61H 7/00, A61M 35/00, B65D 35/36, B65D 47/20, B65D 47/36, B65D 51/24

(54) **CONTAINER CAP AND CONTAINER**

(71) Applicant: Haleon CH SARL, 1197 Prangins (CH)
(72) Inventor: FRADIN, Sylvain Patrice Dominique, 1197 Prangins (CH); ATHAY, Brad, 1197 Prangins (CH)
(74) Representative: Haleon Patent Department

(57) **Abstract**

This disclosure relates to an applicator cap configured to close a container with a receiving space for storing a topical formulation and an opening for dispensing the topical formulation from the receiving space. The applicator cap comprises a cap base body including a connection portion for connecting the applicator cap to a container, to cover an opening of the receiving space of the container, and a lid that is pivotably connected to the cap base body via a hinge and is moveable between a closed position for closing the applicator cap and an open position for opening the applicator cap. The lid comprises a locking portion that is fixable to a mating portion on the cap base body or on the container for a topical formulation, to which the applicator is connected, for fixing the lid in the open position.

## Description

### Technical Field

The present disclosure relates to an applicator cap that closes a container for a topical formulation, such as a cream tube or a gel tube, and to a container comprising such a container cap.

### Technical Background

Containers have been developed which can be used to both store and to dispense topical formulations, such as ointments, gels, creams, and the like. Well known examples of such containers are gel, cream or ointment tubes or bottles.

In addition, application caps have been developed which fit onto a mating part of a container main body, such as a tube, and allow alternatively opening up for dispensing the topical formulation or closing off the container. To this end, applicator heads comprise an outlet channel for expulsion of a product from the container.

In order to increase user convenience, a further development resulted in application caps comprising application surfaces for applying an expelled product onto an application zone, such as a user's skin. Such application surfaces may even comprise surface undulations serving as massage bumps, in order to massage the topical formulation into the skin by moving the application cap. This has the advantage that the topical formulation reaches the target zone and is not partially absorbed by a hand of a user which would otherwise be used to rub in the product.

In addition, further developments serving user convenience have led towards the provision of dosing markings on application surfaces, which allow a user to dispense a certain amount of product, until the product reaches such a dosing marking. This is a very useful feature, as applying an adequate amount of product is often crucial, in order to be able to benefit in a desirable way from a topical formulation, without risking unwanted or excessive side effects, and the like.

Although the developed containers and their applicator caps have greatly increased user convenience, there may nevertheless be drawbacks. For example, a user may inadvertently squeeze out too much of a product while moving the container, in order to rub in the product with a massage head.

Another inconvenience may lie in a user inadvertently getting some product on either or both of the hands while fiddling with the container. When trying to carry out a subsequent action (e.g., dealing with a plaster, a patch, or the like), a user cannot avoid washing the hands prior to continuing, and the like.

The dispensing of topical formulations ought to be precise, easy, intuitive, and clean.

There is, hence, a need for improved containers and/or application heads that address at least one of the above-mentioned shortcomings.

### Summary

Aspects of the above-mentioned object are achieved by an applicator cap in accordance with the present disclosure.

An applicator cap in accordance with the present disclosure may be configured to close a container with a receiving space for storing a topical formulation and an opening for dispensing the topical formulation from the receiving space. The applicator cap may close a container with a receiving space storing a topical formulation and an opening for dispensing the topical formulation from the receiving space.

Particularly, the container may be flexible and a side wall may be squeezed to move contents out of the receiving space of the container. The container may in particular be a cream tube or a gel tube, such as an emulgel tube. In certain embodiments, the container is a flexible container that may be filled and sealed at the base by crimping.

The term "topical formulation" is to be construed broadly in this context and may refer to a formulation including an active ingredient, such as a medication, a drug, or a botanical, as well as a so-called vehicle. The vehicle may comprise or consist of water, oil, alcohol, propylene glycol mixed with preservatives, emulsifiers, absorption promoters, and fragrances, etc. The term topical formulation thus covers any fluid provided with an active ingredient, wherein the fluid is to be dispensed from a container for use. Particularly, the topical formulation has a viscosity greater than that of water (1 cPs), for example a viscosity of at least 100 centipoise (cPs), for example within the range of 100 cPs to 80,000 cPs, such as at least 500 cPs, for example within the range of from 500 cPs to about 80,000 cPs. In certain embodiments the topical formulation has a viscosity of at least 1000 cPs, for example from 1000 cPs to 80,000 cPs. Particular ranges include from 500 cPs to 25,000 cPs, 5000 cPs to 10,000 cPs, 10,000 cPs to 15,000 cPs or 15,000 cPs to 20,000 cPs. Particularly viscosity values may be measured using methods known in the art, for example, using a Brookfield viscometer at a temperature of 25° C.

The topical formulation may be provided in various forms and selected from group comprising gel, emulgel, lotion, cream, oil, emulsion and ointment. Particularly, the topical formulation is a cream, gel or emulgel having a viscosity of at least 100 centipoise. More particularly, the topical formulation is a cream, gel or emulgel having a viscosity of at least 100 centipoise measured using a Brookfield viscometer at a temperature of 25° C. In an embodiment, the topical formulation is a cream, gel or emulgel containing diclofenac sodium, particularly a cream, gel or emulgel containing diclofenac sodium having a viscosity of at least 100 centipoise. In specific embodiments, the topical formulation is a combination of diclofenac sodium, ammonia, carbomer homopolymer type c, cococaprylate/caprate, isopropyl alcohol, mineral oil, polyoxyl 20 cetostearyl ether, propylene glycol, and water.

The applicator cap may be made as a single piece that is connected (i.e., no separable parts, manufactured in one piece). Alternatively, the applicator cap may be made up of two or more separately made parts (i.e. manufactured as two or more separate pieces that are assembled to form the applicator cap) .

In particular, the applicator cap may comprise a cap base body including a connection portion for connecting the applicator cap to a container, to cover an opening of the receiving space of the container.

The applicator cap may comprise a lid that is pivotably connected to the cap base body via a hinge and is moveable between a closed position for closing the applicator cap and an open position for opening the applicator cap.

The cap base body may comprise an application surface and an output channel, connecting an interior space of the cap base body with the application surface and for outputting a topical formulation onto the application surface, to let the topical formulation spread on the application surface.

When the topical formulation is output from the output channel, it may, hence, reach the application surface and may start spreading on the application surface. Optionally, the geometry of the application surface may be chosen symmetrically around an output channel opening, so that the topical formulation spreads out radially from the output channel opening. In other words, the topical formulation may start forming a more or less round dot of topical formulation which starts growing radially around the output channel opening when more topical formulation is output.

The application surface may comprise a massage portion. The massage portion may be a smooth surface or may comprise protrusions and/or recessions that serve to rub the topical formulation into a target zone brought into contact with the massage portion while moving the cap base body. The target zone may, e.g., be a piece of skin with which the user makes contact to apply the topical formulation via the application surface.

The outputting of the topical formulation may be achieved by exerting pressure from the inside. For example, a user may apply pressure to the container, so that the topical formulation is squeezed out of the output channel and onto the application surface.

The massage portion may, e.g., extend circularly around an output channel opening and/or comprise several output channel openings.

According to some embodiments, the massage portion may comprise only protrusions that extend beyond a base surface of the application surface. According to some embodiments, the massage portion may comprise only recessions that appear as indentations with respect to a base surface of the application surface. According to some embodiments, the massage portions may comprise at least one protrusion and at least one recession. According to other embodiments, the massage portion may be a substantially smooth surface having no grooves, protrusions, or the like, having a surface roughness (Ra) of less than or equal to 0.25 microns, for example measured using a profilometer. According to some embodiments, the massage portion may comprise an undulating surface.

The lid may comprise a locking portion that is fixable to a mating portion on the cap base body or on the container for a topical formulation, to which the applicator is connected, for fixing the lid in the open position.

With the lid fixed in the open position, the application of the topical formulation may be much more convenient. The lid may not get in the way, when the topical is being applied. Especially in combination with using a massage portion, the fixing of the lid with the locking portion may be particularly beneficial, as the cap may be moved, the massage portion may thus be moved and the topical formulation be rubbed in, without the risk of the lid getting the way (e.g., snapping back, scratching the skin's surface or inadvertently getting into contact with the topical formulation.

The locking portion may thus also prevent the lid from being contaminated with the topical formulation and in this way contribute to the overall hygiene. A container with the cap may in this way be kept clean. In fact, a user may not even need to wash the hands after using the container with the cap. In this manner a user can be confident that the correct amount of topical formulation has been applied in the correct place without wastage. Surprisingly, avoiding the need for a user to wash their hands also reduces the amount of active ingredient of a topical formulation that might end up in wastewater and water bodies.

The locking portion may comprise at least one protruding part. According to some embodiments, the locking portion may comprise exactly one protruding part.

The locking portion may comprise at least two protruding parts.

According to some embodiments, the locking portion may comprise exactly two protruding parts.

The locking portion may comprise at least three or more protruding parts. The locking portion may comprise exactly three or more protruding parts.

The at least one protruding part may be configured to, upon the lid being hinged into the open position, to engage with the mating portion to prevent the lid from inadvertently moving back towards the closed position.

The cap base body may comprise the mating portion and the mating portion may comprise a protruding portion against which the at least one protruding part rests to prevent a movement of the lid when fixed in the open position. This may be a convenient mechanical construction which may be manufacturable at reasonably low cost.

The massage portion may partially surround the output channel so that topical formulation output from the output channel spreads over the massage portion.

The massage portion may fully surround the output channel so that topical formulation output from the output channel spreads over the massage portion.

The application surface may comprise a dosage marking portion. Optionally, the massage portion may comprise the dosage marking portion. According to some embodiments, the dosage marking portion may be the outer boundary of the massage portion.

The dosage marking portion may be a great way to help users output the right amount of the topical formulation from the container. The user may in particular output topical formulation until the topical formulation has spread out roughly up to the dosing making portion. Put differently, a user can be aided in dosing the topical formulation correctly by being instructed to output topical formulation until it reaches the marking portion upon spreading on the application surface.

The dosing marking portion may be a dosing ring marking. In other words, the marking portion may have a ring shape or a shape of a part of ring, or it may be partially ring-shaped.

The dosing ring marking may be a circular shaped dosing marking portion.

The dosing marking portion may be arranged concentrically around the output channel.

The dosage marking portion may surround the output channel at a predetermined distance and indicate a predetermined amount of a topical formulation by virtue of being reached by a topical formulation spreading on the application surface.

The application surface may comprise two or more dosage marking portions. In particular, the application surface may, e.g., if there are two or more output channels, comprise at least one dosage marking portion for each of the two or more output channels.

A single output channel (which may be the only output channel) may be provided with two or more dosage marking portions. This way, more than one dosage amount may be indicated to a user.

The massage portion may comprise an inner massage portion that is surrounded by the dosing marking portion and an outer massage portion that surrounds the dosing marking portion. The inner and outer massage portions may both help in massaging in a topical formulation on a target zone, as the topical formulation may, upon moving around the cap with respect to the target zone, also spread out further than up to the dosing marking portion.

The inner massage portion may surround the output channel, and it may in particular surround it concentrically.

The massage portion may comprise at least one hexagonal protrusion.

The massage portion may comprise a honey-comb arrangement of hexagonal protrusions. This may provide a particularly efficient massage / rubbing-in functionality.

According to other embodiments, the protrusions may have different shapes (e.g., circular, polygonal with other amounts of corners, oval, etc.).

The cap base body may comprise a connector part that is connectable to the container. The connector part may thus provide an interface with respect to a container / container base body which stores a topical formulation.

The connector part may be screwed onto a container / container base body, or it may be connected therewith in a different manner (press fit / friction engagement, etc.). In some embodiments, the connector part may comprise a tamper evidence means for providing evidence of opening. In some embodiments, the connector part comprises tamper-proof closure means for locking the applicator cap to the container to prevent contamination or tampering with the contents.

The cap base body may comprise a moveable tip part which comprises the application surface and which is moveable relative to the connector part between a locking state in which the output channel is shut off from dispensing and a dispensing state in which the output channel communicates between an interior of the case base body and the application surface for dispensing a topical formulation.

By moving the tip part with respect to the connector part, the output channel may thus be opened or closed.

Alternatively, the cap base body may comprise a third part, and the moveable tip part may be moveable relative to the third part between a locking state in which the output channel is shut off from dispensing and a dispensing state in which the output channel communicates between an interior of the case base body and the application surface for dispensing a topical formulation.

The moveable tip part may comprise a catch that is engaged by the lid upon opening the lid from the closed state, wherein an initial opening movement of the lid may draw the moveable tip part into the dispensing state. Such embodiments may be particularly convenient in that a user does not need to do anything to arrive at the dispensing state. This may be automated by the engagement of the moveable tip part.

The moveable tip part may be moveable from the locked state to the dispensing state by a user pulling the moveable tip part away from the connector part.

However, for other embodiments, it may be preferable for the user to be able to determine when the output channel is opened, and such embodiments may not comprise a catch as described above, and, more generally, may not have a mechanism permitting the moveable tip part to be moved into a dispensing state by moving the lid. For such embodiments, the user may perform a movement to bring the dispensing channel into the open state (e.g., by pulling a moveable part manually and, to close it, by then pushing it back shut).

The moveable tip part may (be configured to) move back from the dispensing state to the locking state pressure being applied to the application surface. According to some embodiments, the lid may apply a sufficient pressure to move the moveable tip part back into the locking state when closing the lid. According to other embodiments, the use may need to push back the moveable tip part.

The moveable tip part may be translatable in a longitudinal direction of the applicator cap. The longitudinal direction may be parallel to a central axis extending from a connector side to a dispensing side of the applicator cap. When the applicator cap is placed on a tube, the longitudinal direction may also be the longitudinal direction of the container (e.g., a tube).

A part of the output channel provided on the moveable tip part fluid may, in the locked, state tightly sit on a rest seat surface that shuts off the inner end of the output channel. This may be an easy construction for blocking the output in the locked state.

The rest seat surface may be provided on a protruding rod on the connector part of the cap base body.

As mentioned above, the application surface may in each case (that is, combined with any one or several of the features mentioned) comprise one output channel or a plurality of output channels.

The cap base body may comprise a front wall that forms an angle of from 60° to 90° with respect to a longitudinal direction of the cap base body. In other words, the front wall may be orthogonal to the longitudinal direction. However, it may also be slanted, as this may be convenient for applying a topical formulation.

The cap base body may comprise a front wall that forms an angle of from 70° to 80° with respect to a longitudinal direction of the cap base body.

The front wall may comprise the application surface.

The output channel may penetrate the front wall.

The front wall may comprise a ridge portion on an inside thereof which may surround an inside end of the output channel.

The cap base body may comprise a thread engagement portion for screwing the cap onto a mating thread engagement portion of a container.

The lid may be connected to the cap base body through at least one plastically deformable bridge portion that serves as a hinge. The hinge may allow for a particularly convenient movement of the lid namely, by hinging the lid back and forth to open and close the container bay opening and closing the lid of the container cap.

The lid may be hingeable around a hinge axis direction by at least 150°.

The lid may be hingeable around a hinge axis direction by at least 170°, at least 180°, or at least 190°, at least 200°, or at least 210°.

The lid may comprise at least one material selected from the group consisting of silicon, LDPE, TPE, PE, PP, rubber, and PET. It may comprise one of the mentioned materials.

The lid may consist of at least one material selected from the group consisting of silicon, LDPE, TPE, PE, PP, rubber, and PET. It may consist of one of the mentioned materials.

The container cap may comprise a tamper evidence means for providing evidence of first opening of the applicator cap. For example, a tamper evidence mechanism comprising at least one frangible part that breaks upon the lid being opened from the closed state to the open state for the first time. In certain embodiments, the tamper evidence means may be a frangible adhesive label or plastic film over-wrap. Tamper evidence means may provide consumer safety.

At least a part of the tamper evidence mechanism may be at least in part provided on a rim of the lid.

The lid may cover the output channel in the closed position and reveal the output channel in the open position.

The lid may cover at least a part of the application surface in the closed position and reveal the application surface in the open position.

The lid may cover the entire application surface in the closed position and reveal the application surface in the open position.

The applicator surface may comprise a central flat portion. and an outer rounded circumference portion surrounding the central flat portion.

According to some embodiments, protrusions protrude and/or recessions recede from the central flat portion.

The present disclosure also relates to a container. The container may comprise a base body with a receiving space that may receive a topical formulation, such as a cream, gel or emulgel, and the container may comprise a container cap in accordance with any one or several embodiments described above. In particular, the container cap may have any one or several of the features of containers caps described above, in as far as the features are not mutually contradictive. The container cap may be connected or connectable to the container.

The container may be deformable so that a user can press it from the outside to apply pressure and in this way dispense topical formulation from the output channel of the container cap and onto the application surface (in particular, the massage surface).

The container may comprise a mating portion, wherein the mating portion may comprise a protruding portion against which the at least one protruding part rests to prevent a movement of the lid when fixed in the open position.

Additional advantages and features of the present disclosure, that can be realized on their own or in combination with one or several features discussed above, insofar as the features do not contradict each other, will become apparent from the following embodiments and description.

### Specific Embodiments

EMBODIMENT 1: An applicator cap configured to close a container with a receiving space storing a topical formulation, such as a cream tube or a gel tube, and an opening for dispensing the topical formulation from the receiving space, the applicator cap comprising: a cap base body including a connection portion for connecting the applicator cap to a container, to cover an opening of the receiving space of the container, and a lid that is pivotably connected to the cap base body via a hinge and is moveable between a closed position for closing the applicator cap and an open position for opening the applicator cap, wherein the cap base body comprises an application surface and an output channel, connecting an interior space of the cap base body with the application surface and for outputting a topical formulation onto the application surface, to let the topical formulation spread on the application surface, wherein the application surface comprises a massage portion that serves to rub the topical formulation into a target zone brought into contact with the massage portion while moving the cap base body, wherein the lid comprises a locking portion that is fixable to a mating portion on the cap base body or on the container for a topical formulation, to which the applicator is connected, for fixing the lid in the open position.

EMBODIMENT 2: The applicator cap of EMBODIMENT 1, wherein the locking portion comprises at least one protruding part, optionally two protruding parts, or three or more protruding parts, wherein the at least one protruding part, upon the lid being hinged into the open position, is configured to engage with the mating portion to prevent the lid from inadvertently moving back towards the closed position.

EMBODIMENT 3: The applicator cap of EMBODIMENT 2, wherein the cap base body comprises the mating portion and the mating portion comprises a protruding portion against which the at least one protruding part rests to prevent a movement of the lid when fixed in the open position.

EMBODIMENT 4: The applicator cap of any one of EMBODIMENTS 1 to 3, wherein the massage portion at least partially, optionally fully, surrounds the output channel so that topical formulation output from the output channel spreads over the massage portion.

EMBODIMENT 5: The applicator cap of EMBODIMENT 4, wherein the application surface, optionally the massage portion, comprises a dosage marking portion, such as a dosing ring marking, optionally a circular shaped dosing marking portion, optionally the dosing marking portion being arranged concentrically around the output channel, the dosage marking portion surrounding the output channel at a predetermined distance and indicating a predetermined amount of a topical formulation by virtue of being reached by a topical formulation spreading on the application surface.

EMBODIMENT 6: The applicator cap of EMBODIMENT 5, wherein the massage portion comprises an inner massage portion that is surrounded by the dosing marking portion and an outer massage portion that surrounds the dosing marking portion, wherein the inner massage portion surrounds the output channel.

EMBODIMENT 7: The applicator cap of EMBODIMENT 5 or 6, wherein the massage portion comprises protrusions and/or recessions.

EMBODIMENT 8: The applicator cap of EMBODIMENT 7, wherein the massage portion comprises at least one hexagonal protrusion, the massage portion optionally comprising a honey-comb arrangement of hexagonal protrusions.

EMBODIMENT 9: The applicator cap of any one of the preceding EMBODIMENTS, wherein the cap base body comprises a connector part, connectable to the container, and a moveable tip part, the moveable tip part comprising the application surface and being moveable relative to the connector part between a locking state in which the output channel is shut off from dispensing and a dispensing state in which the output channel communicates between an interior of the case base body and the application surface for dispensing a topical formulation.

EMBODIMENT 10: The applicator cap of EMBODIMENT 9, wherein the moveable tip part comprises a catch that is engaged by the lid upon opening the lid from the closed state, wherein an initial opening movement of the lid draws the moveable tip part into the dispensing state.

EMBODIMENT 11: The applicator cap of EMBODIMENT 9, wherein the moveable tip part is moveable from the locked state to the dispensing state by a user pulling the moveable tip part away from the connector part.

EMBODIMENT 12: The applicator cap of any one of EMBODIMENTS 9 to 11, wherein the moveable tip part moves back from the dispensing state to the locking state pressure being applied to the application surface.

EMBODIMENT 13: The applicator cap of any one of EMBODIMENTS 9 to 12, wherein the moveable tip part is translatable in a longitudinal direction of the applicator cap, and, wherein, in the locked state, a part of the output channel provided on the moveable tip part fluid tightly sits on a rest seat surface that shuts off the inner end of the output channel, the rest seat surface optionally being provided on a protruding rod on the connector part of the cap base body.

EMBODIMENT 14: The applicator cap of any one of the preceding EMBODIMENTS, wherein the application surface comprises one output channel or a plurality of output channels.

EMBODIMENT 15: The applicator cap of any one of the preceding EMBODIMENTS, wherein the cap base body comprises a front wall that forms an angle of from 60° to 90°, optionally from 70° to 80°, with respect to a longitudinal direction of the cap base body, wherein the front wall comprises the application surface, and the output channel penetrates the front wall.

EMBODIMENT 16: The applicator cap of EMBODIMENT 15, wherein the front wall comprises a ridge portion on an inside thereof which surrounds an inside end of the output channel.

EMBODIMENT 17: The applicator cap of any one of the preceding EMBODIMENTS, wherein the cap base body comprises a thread engagement portion for screwing the cap onto a mating thread engagement portion of a container.

EMBODIMENT 18: The applicator cap of any one of the preceding EMBODIMENTS, wherein the lid is connected to the cap base body through at least one plastically deformable bridge portion that serves as a hinge.

EMBODIMENT 19: The applicator cap of any one of the preceding EMBODIMENTS, wherein the lid is hingeable around a hinge axis direction by at least 150°, optionally at least 170°, at least 180°, or at least 190°, at least 200°, or at least 210°.

EMBODIMENT 20: The applicator cap of any one of the preceding EMBODIMENTs, comprising at least one material selected from the group consisting of silicon, LDPE, TPE, PE, PP, rubber, and PET.

EMBODIMENT 21: The applicator cap of any one of the preceding EMBODIMENTs, comprising a tamper evidence mechanism including a frangible part that breaks upon the lid being opened from the closed state to the open state for the first time.

EMBODIMENT 22: The applicator cap of EMBODIMENT 21, wherein at least a part of the tamper evidence mechanism is at least in part provided on a rim of the lid.

EMBODIMENT 23: The applicator cap of any one of the preceding EMBODIMENTS, wherein the lid covers the output channel in the closed position and reveals the output channel in the open position, and, optionally, the lid covers at least a part of, optionally the entire, application surface in the closed position and reveals the application surface in the open position.

EMBODIMENT 24: The applicator cap of any one of the preceding EMBODIMENTS, wherein the applicator surface comprises a central flat portion and an outer rounded circumference portion surrounding the central flat portion, wherein, optionally, protrusions protrude and/or recessions recede from the central flat portion.

EMBODIMENT 25: A container comprising: a base body with a receiving space that receives a topical formulation, such as a cream or gel, and the container cap according to any one of the preceding EMBODIMENTS, wherein the container cap is connected to the container.

EMBODIMENT 26: The container of EMBODIMENT 25, comprising the mating portion, wherein the mating portion comprises a protruding portion against which the at least one protruding part rests to prevent a movement of the lid when fixed in the open position.

### Brief Description of the Drawings

For a better understanding of the present disclosure and to show how the same may be carried into effect, reference will now be made, by way of example only, to the accompanying drawings, in which:
The description is given with reference to the accompanying drawings, in which:
- Fig. 1: is a perspective view of an applicator cap in accordance with an embodiment of the present disclosure;
- Fig. 2: is a side view of an applicator cap in accordance with an embodiment of the present disclosure;
- Fig. 3: is a bottom view of an applicator cap in accordance with an embodiment of the present disclosure;
- Fig. 4: depicts a side view of a lid of an applicator cap in accordance with an embodiment of the present disclosure;
- Fig. 5: depicts a part of an applicator cap in accordance with an embodiment of the present disclosure without a lid;
- Fig. 6: is a top view onto an inside of an applicator cap in accordance with an embodiment of the present disclosure without a lid;
- Fig. 7: is a top view of a connection part of an applicator cap in accordance with an embodiment of the present disclosure for establishing a connection with a container;
- Fig. 8: is a perspective view of a connection part of an applicator cap in accordance with an embodiment of the present disclosure for establishing a connection with a container;
- Fig. 9: is a perspective view of a part of an applicator cap in accordance with an embodiment of the present disclosure with an applicator surface;
- Fig. 10: is a perspective bottom view of a part of an applicator cap in accordance with an embodiment of the present disclosure with an applicator surface;
- Fig. 11: a side view of a container with a container cap in accordance with an embodiment of the present disclosure;
- Fig. 12: a perspective view of a container with a container cap in accordance with an embodiment of the present disclosure;
- Fig. 13: a perspective view of a container with a container cap in accordance with an embodiment of the present disclosure without a lid;
- Fig. 14: is a side view of an applicator cap in accordance with an embodiment of the present disclosure;
- Fig. 15: is a bottom perspective view of an applicator cap in accordance with an embodiment of the present disclosure; and
- Fig. 16: is a bottom perspective view of the lid of the applicator cap of Fig. 15.

### Detailed Description

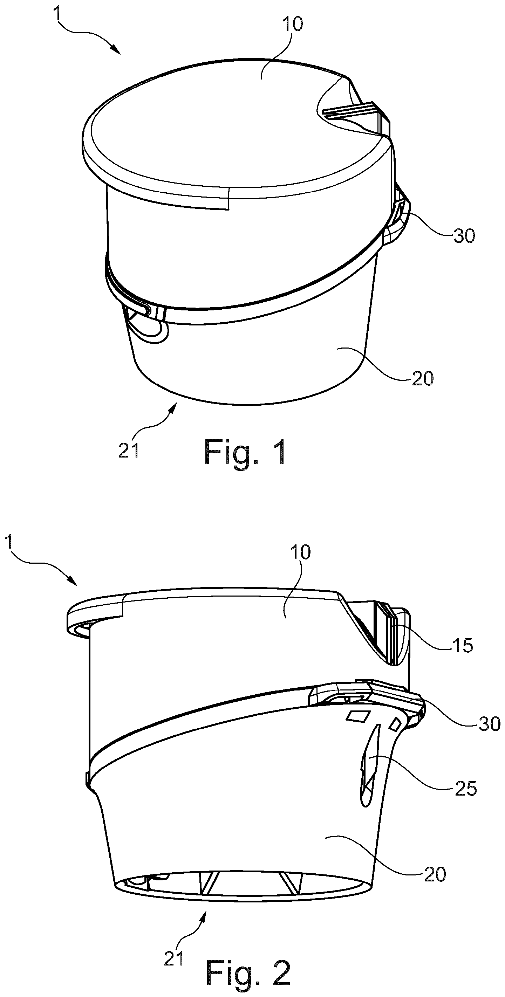

Fig. 1 is a perspective view of of an applicator cap 1 in accordance with an embodiment of the present disclosure.

The applicator cap 1 is for closing a container (not shown in Fig. 1) with a receiving space storing a topical formulation, such as a cream tube or a gel tube, and an opening (not shown in Fig. 1, as it is covered by a lid 10) for dispensing the topical formulation from the receiving space.

There are different versions of the applicator cap 1 of Fig. 1, namely, versions, for example, comprising or consisting of at least one material selected from the group consisting of silicon, LDPE, TPE, PE, PP, rubber, and PET.

The applicator cap 1 comprises a cap base body 20 including a connection portion 21 for connecting the applicator cap 1 to a container, to cover an opening of the receiving space of the container so that the topical formulation is shielded.

The lid 10 is pivotably connected to the cap base body 20 via a hinge 30 and is moveable between a closed position for closing the applicator cap 1 and an open position for opening the applicator cap 1.

The lid 10 is hingeable around a hinge axis direction by at least 150° in the case of this embodiment. This way, the lid 10 may be hinged far enough to be able to make contact with the locking portion 25 to fix the lid 10 in the open position.

Fig. 2 shows a side view of the applicator cap 1 of Fig. 1. It can be seen in Fig. 2 that the bottom side of the applicator cap 1 is open and that a connection of the connection portion 21 with a container will thus expose an upper side of the container to the inside of the applicator cap 1.

The lid 10 comprises a locking portion 15 that is fixable to a mating portion 25 on the cap base body 20 for fixing the lid 1 in the open position. Thus, by hinging back the lid 10, the locking portion 15 can be pushed to fasten with the mating portion 25.

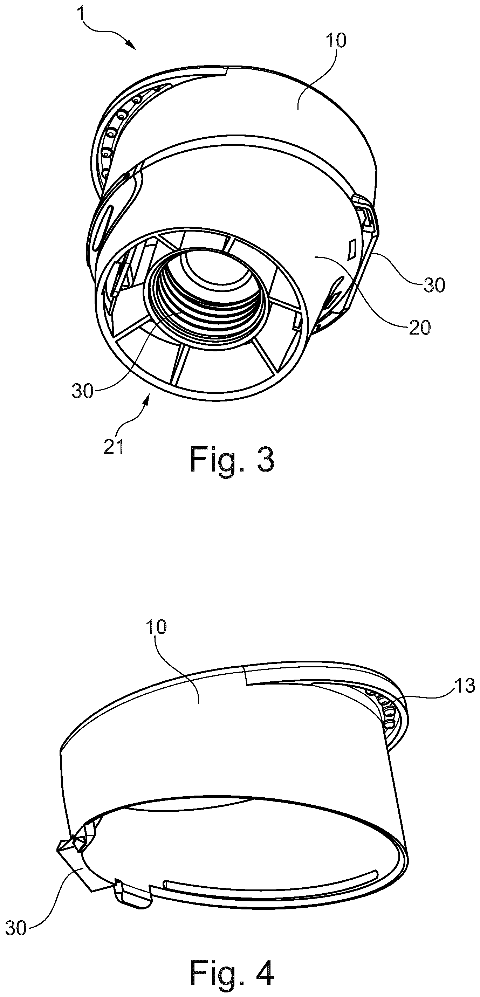

Fig. 3 shows a bottom view of the applicator cap 1 of Fig. 1. The cap base body 20 comprises a thread engagement portion 22 for screwing the cap onto a mating thread engagement portion of a container.

Fig. 4 shows a perspective view of the lid 10 only of the applicator cap 1 of Figs. 1-3.

The lid 10 has an engagement portion 13 which allows a user to easily engage and move the lid 10 with a finger. A part of the hinge 30 is also visible in Fig. 4, as it may be considered part of the lid 10.

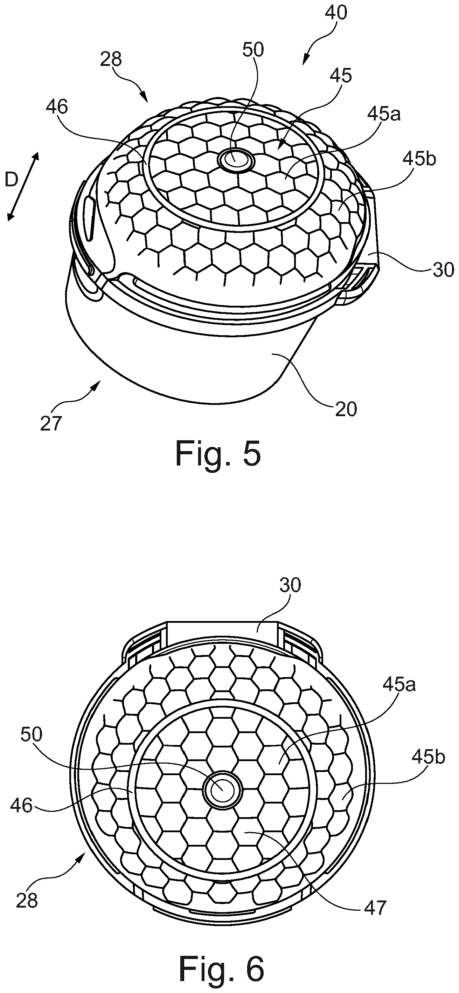

Looking at Figs. 4 and 5 simultaneously, it is to be understood that the lid 10 covers the output channel 50 in the closed position and reveals the output channel 50 in the open position, and, in the case of this embodiment, the lid 10 also covers the entire application surface 40 in the closed position and reveals the application surface 40 in the open position.

Fig. 5 shows a perspective view of an embodiment of the applicator cap 1 in accordance with the present disclosure, but without a lid, in order to expose what lies beneath in the closed state.

The base body 20 comprises an upper portion (which is covered by the lid 10 in Figs. 1-3) which comprises an application surface 40 and an output channel 50, connecting an interior space of the cap base body 20 with the application surface 40 and for outputting a topical formulation onto the application surface 40, to let the topical formulation spread on the application surface 40.

The application surface 40 comprises a massage portion 45 comprising protrusions that serve to rub the topical formulation into a target zone (e.g., a skin surface) brought into contact with the massage portion 45 while moving the cap base body 20.

The massage portion 45 in fact comprises a dosage marking portion 46, in this case with a ring-shape, being arranged concentrically around the output channel 50. The dosage marking portion 46 surrounds the output channel 50 at a predetermined distance and indicates a predetermined amount of a topical formulation by virtue of being reached by a topical formulation spreading on the application surface 40.

The massage portion 45 comprises an inner massage portion 45a that is surrounded by the dosing marking portion 46 and an outer massage portion 45b that surrounds the dosing marking portion 46. The inner massage portion 45a and the outer massage portion 45b both concentrically surround the output channel 50. This is also visible in Fig. 6, showing a top view of the base body 20 of Fig. 5. A part of the hinge 30 is also visible in Figs. 5 and 6.

The massage portion 45 fully surrounds the output channel 50 so that topical formulation output from the output channel 50 homogeneously spreads over the massage portion 45 (at least if the container and the cap are held more or less upright).

The massage portion 45 comprises hexagonal protrusions 47. The hexagonal protrusions 45 are together arranged to form a honey-comb arrangement.

More generally, the applicator surface 40 comprises a central flat portion and an outer rounded circumference portion surrounding the central flat portion, wherein the hexagonal protrusions 47 protrude from the central flat portion, and there are some further protrusions 47 at the rounded circumference portion. The outer part of the rounded circumference portion is smooth and comprises no protrusions or recessions.

The output channel 50 is shown in the closed state in Figs. 5 and 6. In other words, the part of the application surface 40 is pushed down, so that no space for any topical formulation is left to exit the output channel 50.

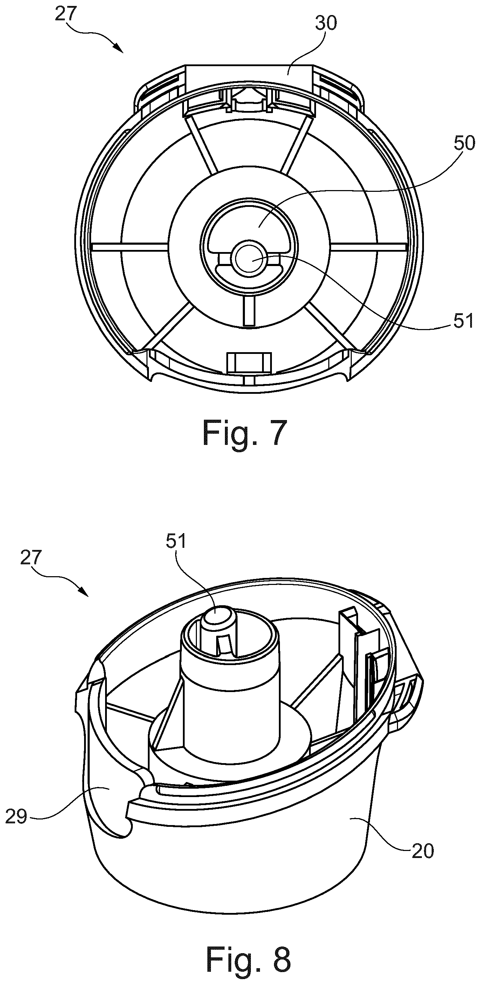

Fig. 7 shows a top view of a part of the base body 20, without the upper part including the application surface 40.

In Fig. 7, a part of the output channel 50 is visible. It surrounds a protruding part 51, which blocks the output channel 50 when the upper part (shown in Figs. 5 and 6 but omitted in Fig. 7) is pushed downwards, so that it rests against the part shown in Fig. 7.

Fig. 8 shows a perspective view of the same part of the base bod 20 that is also shown in Fig. 7. The protruding part 51 that is configured to shut off the output channel 50 in the closed state protrudes the furthest upward in Fig. 8. The output channel 50 is surrounded by a circumferential wall 52 below the protruding part 51..

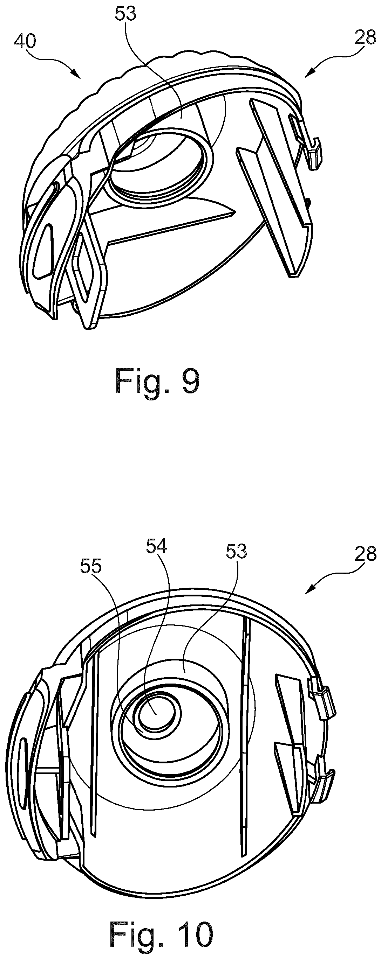

Fig. 9 shows a perspective view from a lower position of the upper part of the base body 20 of Figs. 5 and 6.

The applicator surface 40 is on the top side and is only visible a bit in Fig. 9. Another circumferential wall part 53 is shown in Fig. 9 which surrounds the output channel 50.

The part of the base body 20 of the applicator cap 1 shown in Figs. 7 and 8 and, e.g., at the bottom side of Fig. 5 is also referred to as a connector part 27, connectable to a container.

The part of the base body 20 of the applicator cap 1 shown in Fig. 9 and, e.g., at the top side of Fig. 5 as well as in Fig. 6, is also referred to as a moveable tip part 28.

The moveable tip part 28 comprises the application surface 40 (se Fig. 9) and being moveable relative to the connector part 27 between a locking state in which the output channel 50 is shut off from dispensing and a dispensing state in which the output channel 50 communicates between an interior of the case base body 20 and the application surface 40 for dispensing a topical formulation.

The moveable tip part 28 moves back from the dispensing state to the locking state by pressure being applied to the application surface 40. This is, e.g., done by closing the lid 10. The lid 10 then exerts pressure on the moveable tip part 28 so that it is pressed back and so that the output channel 50 is closed.

In the case of this embodiment, the moveable tip part 28 is moveable from the locked state to the dispensing state by a user pulling the moveable tip part 28 away from the connector part 27. To this end, the user can press a release part 29 (see Fig. 8) on the connector part 27 to disengage the moveable tip part 28.

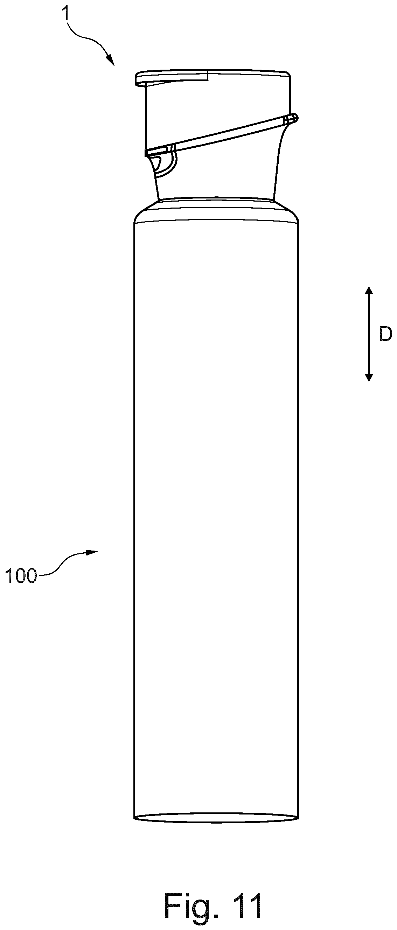

The moveable tip part 28 is in the case of this embodiment translatable in a longitudinal direction D (see Figs. 5 and 11) of the applicator cap 1 (which also happens to be the longitudinal direction of the container 100).

Fig. 10 shows a bottom view of the moveable tip part 28 of Fig. 9. Fig. 10 shows the opening 54 of the output channel 50 to the application surface (on the side of the moveable tip part 28 facing away in Fig. 10).

A rim of a part of the output channel 50 leads up to the opening 54. This part of the output channel 50, also referred to as a ridge portion 55, in the locked state, fluid tightly sits on a rest seat surface that shuts off the inner end of the output channel. This rest seat surface is provided by the protruding rod 51 on the connector part 27 of the cap base body 20 (see, e.g., Figs. 7 and 8).

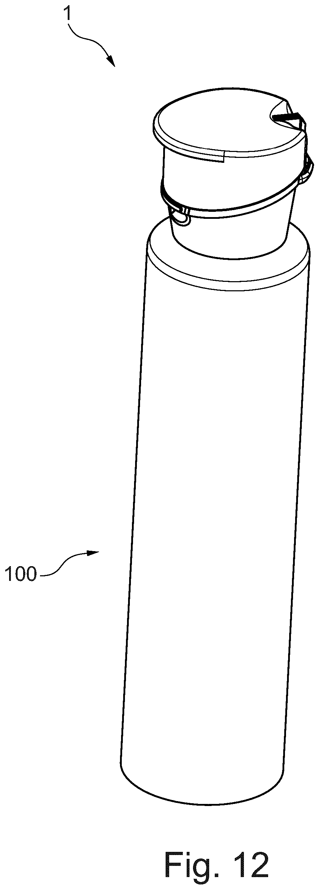

Fig. 11 shows a side view of a container 100 provided with the container cap 1 discussed in the context of Figs. 1-9. The container 100 of Fig. 11 is in fact a tube, but it has not yet been welded closed at the lower end. That is, the state in Fig. 12 is prior to filling the container 100 with a topical formulation and to closing it off for sealing it at the lower end.

Fig. 12 shows a perspective view of a container 100 provided with the container cap 1 discussed in the context of Figs. 1-9. The container 100 of Fig. 11 is in fact a tube, but it has not yet been welded closed at the lower end. That is, the state in Fig. 12 is prior to filling the container 100 with a topical formulation and to closing it off for sealing it at the lower end.

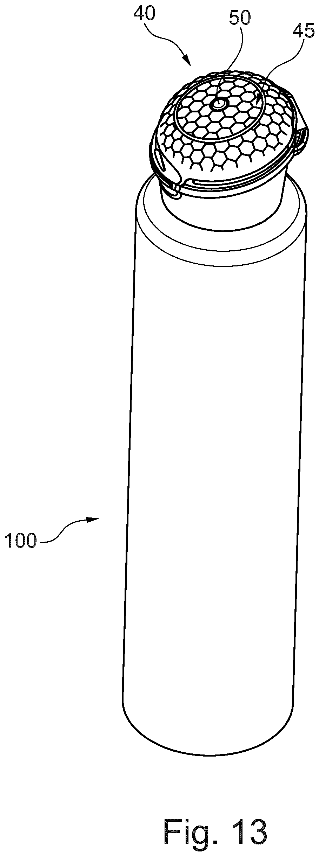

Fig. 13 shows a perspective view of the container 100 of Figs. 11 and 11, but the lid is omitted. Thus, the application surface 40 with the massage portion 45 and the output channel 50 in the middle is visible.

The front wall (at the top of Fig. 13) of the cap base body 20 which comprises the application surface 40 forms an angle in the range of 60° to (less than) 90° with respect to the longitudinal direction of the cap base body 20. The output channel 50 penetrates said front wall.

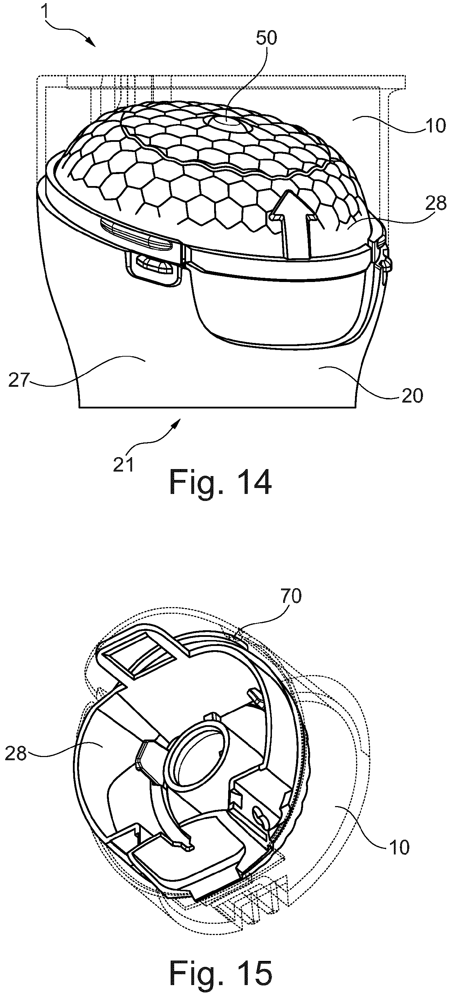

Fig. 14 is a perspective view of of an applicator cap 1 in accordance with an embodiment of the present disclosure. In many aspects, it is similar to the embodiment shown in Fig. 1.

The applicator cap 1 of Fig. 14 is for closing a container (not shown in Fig. 10) with a receiving space storing a topical formulation, such as a cream tube or a gel tube, and an opening (not shown in Fig. 10, as it is covered by a lid 10) for dispensing the topical formulation from the receiving space.

The applicator cap 1 comprises a cap base body 20 including a connection portion 21 for connecting the applicator cap 1 to a container, to cover an opening of the receiving space of the container so that the topical formulation is shielded.

The lid 10 is pivotably connected to the cap base body 20 via a hinge and is moveable between a closed position for closing the applicator cap 1 and an open position for opening the applicator cap 1.

The lid 10 is hingeable around a hinge axis direction by at least 150° in the case of this embodiment. This way, the lid 10 may be hinged far enough to be able to make contact with the locking portion 25 to fix the lid 10 in the open position.

The lid 10 comprises a locking portion that is fixable to a mating portion on the cap base body 20 for fixing the lid 1 in the open position. Thus, by hinging back the lid 10, the locking portion can be pushed to fasten with the mating portion.

The base body 20 of the applicator cap 1 of Fig. 14 comprises a connector part 27 for connecting the applicator cap 1 to a container (not shown) and a moveable tip part 28 that is translatable in the longitudinal direction of the applicator cap 1 with respect to the connector part 27, to open the output channel 50.

What is important with respect to the embodiment of Fig. 14, is that the opening of the lid 10 automatically also pulls the moveable tip part 28 away from the connector part 27, so that the opening of the lid 10 automatically also frees the output channel 50 for dispensing.

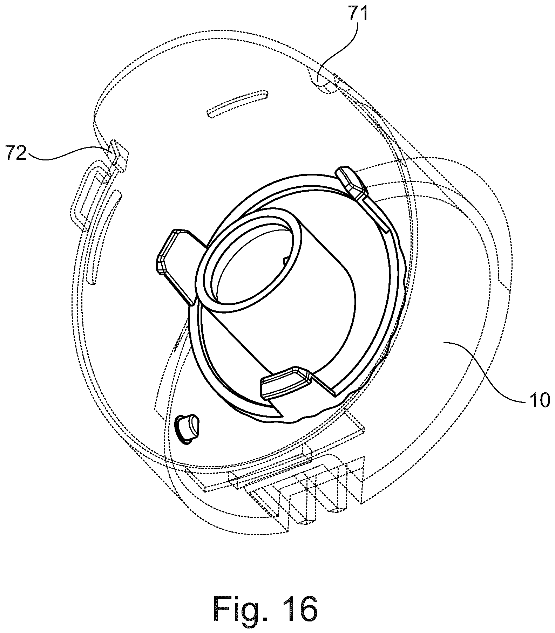

Fig. 15 shows a bottom side view of a part of the applicator cap 1 of Fig. 14, but with the connector part 27 removed, to expose the view from below onto the moveable tip part 28 and the lid 10. Fig. 16 shows the lid 10 without the moveable tip part 28.

The moveable tip part 28 comprises a catch 70 (see Fig. 15) that is engaged by the lid 10 upon opening the lid 10 from the closed state, wherein an initial opening movement of the lid 10 draws the moveable tip part 28 into the dispensing state. To engage with the catch 70, the lid 10 of this embodiment comprises a first protrusion 71 and a second protrusion 72 (see Fig. 16) protruding from an inside surface.

It will be apparent to those skilled in the art that various modifications and variations can be made in the disclosed devices and systems without departing from the scope of the disclosure. Other aspects of the disclosure will be apparent to those skilled in the art from consideration of the specification and practice of the features disclosed herein. It is intended that the specification and examples be considered as exemplary only. Many additional variations and modifications are possible and are understood to fall within the framework of the disclosure.

## Claims

1. An applicator cap configured to close a container with a receiving space storing a topical formulation, such as a cream tube or a gel tube, and an opening for dispensing the topical formulation from the receiving space,
the applicator cap comprising:
a cap base body including a connection portion for connecting the applicator cap to a container, to cover an opening of the receiving space of the container, and
a lid that is pivotably connected to the cap base body via a hinge and is moveable between a closed position for closing the applicator cap and an open position for opening the applicator cap,
wherein the cap base body comprises an application surface and an output channel, connecting an interior space of the cap base body with the application surface and for outputting a topical formulation onto the application surface, to let the topical formulation spread on the application surface,
wherein the application surface comprises a massage portion that serves to rub the topical formulation into a target zone brought into contact with the massage portion while moving the cap base body,
wherein the lid comprises a locking portion that is fixable to a mating portion on the cap base body or on the container for a topical formulation, to which the applicator is connected, for fixing the lid in the open position.

2. The applicator cap of claim 1,
wherein the locking portion comprises at least one protruding part, optionally two protruding parts, or three or more protruding parts,
wherein the at least one protruding part, upon the lid being hinged into the open position, is configured to engage with the mating portion to prevent the lid from inadvertently moving back towards the closed position.

3. The applicator cap of claim 2,
wherein the cap base body comprises the mating portion and the mating portion comprises a protruding portion against which the at least one protruding part rests to prevent a movement of the lid when fixed in the open position.

4. The applicator cap of any one of claims 1 to 3,
wherein the massage portion at least partially, optionally fully, surrounds the output channel so that topical formulation output from the output channel spreads over the massage portion.

5. The applicator cap of claim 4,
wherein the application surface, optionally the massage portion, comprises a dosage marking portion, such as a dosing ring marking, optionally a circular shaped dosing marking portion, optionally the dosing marking portion being arranged concentrically around the output channel,
the dosage marking portion surrounding the output channel at a predetermined distance and indicating a predetermined amount of a topical formulation by virtue of being reached by a topical formulation spreading on the application surface.

6. The applicator cap of claim 5,
wherein the massage portion comprises an inner massage portion that is surrounded by the dosing marking portion and an outer massage portion that surrounds the dosing marking portion,
wherein the inner massage portion surrounds the output channel.

7. The applicator cap of claim 5 or 6, wherein the massage portion comprises protrusions and/or recessions.

8. The applicator cap of claim 7, wherein the massage portion comprises at least one hexagonal protrusion, the massage portion optionally comprising a honey-comb arrangement of hexagonal protrusions.

9. The applicator cap of any one of the preceding claims,
wherein the cap base body comprises a connector part, connectable to the container, and a moveable tip part, the moveable tip part comprising the application surface and being moveable relative to the connector part between a locking state in which the output channel is shut off from dispensing and a dispensing state in which the output channel communicates between an interior of the case base body and the application surface for dispensing a topical formulation.

10. The applicator cap of claim 9,wherein:
(a) the moveable tip part comprises a catch that is engaged by the lid upon opening the lid from the closed state, wherein an initial opening movement of the lid draws the moveable tip part into the dispensing state; or
(b) the moveable tip part is moveable from the locked state to the dispensing state by a user pulling the moveable tip part away from the connector part.

11. The applicator cap of any one of claims 9 to 10, wherein the moveable tip part moves back from the dispensing state to the locking state pressure being applied to the application surface.

12. The applicator cap of any one of claims 9 to 11, wherein the moveable tip part is translatable in a longitudinal direction of the applicator cap, and,
wherein, in the locked state, a part of the output channel provided on the moveable tip part fluid tightly sits on a rest seat surface that shuts off the inner end of the output channel, the rest seat surface optionally being provided on a protruding rod on the connector part of the cap base body.

13. The applicator cap of any one of the preceding claims, wherein (a) the application surface comprises one output channel or a plurality of output channels; and/or (b) the cap base body comprises a front wall that forms an angle of from 60° to 90°, optionally from 70° to 80°, with respect to a longitudinal direction of the cap base body,
wherein the front wall comprises the application surface, and
the output channel penetrates the front wall.

14. The applicator cap of claim 13, wherein the front wall comprises a ridge portion on an inside thereof which surrounds an inside end of the output channel.

15. The applicator cap of any one of the preceding claims, wherein
(a) the cap base body comprises a thread engagement portion for screwing the cap onto a mating thread engagement portion of a container; and/or
(b) the lid is connected to the cap base body through at least one plastically deformable bridge portion that serves as a hinge; and/or
(c) the lid is hingeable around a hinge axis direction by at least 150°, optionally at least 170°, at least 180°, or at least 190°, at least 200°, or at least 210°; and/or
(d) the applicator cap comprises at least one material selected from the group consisting of silicon, LDPE, TPE, PE, PP, rubber, and PET; and/or
(e) the applicator cap comprises a tamper evidence mechanism including a frangible part that breaks upon the lid being opened from the closed state to the open state for the first time.

16. The applicator cap of claim 15, wherein at least a part of the tamper evidence mechanism is at least in part provided on a rim of the lid.

17. The applicator cap of any one of the preceding claims, wherein the lid covers the output channel in the closed position and reveals the output channel in the open position, and, optionally, the lid covers at least a part of, optionally the entire, application surface in the closed position and reveals the application surface in the open position.

18. The applicator cap of any one of the preceding claims, wherein the applicator surface comprises a central flat portion and an outer rounded circumference portion surrounding the central flat portion, wherein, optionally, protrusions protrude and/or recessions recede from the central flat portion.

19. A container comprising:
a base body with a receiving space that receives a topical formulation, such as a cream or gel, and
the container cap according to any one of the preceding claims, wherein the container cap is connected to the container.

20. The container of claim 19, comprising the mating portion, wherein the mating portion comprises a protruding portion against which the at least one protruding part rests to prevent a movement of the lid when fixed in the open position.
